# EUROPÄISCHE PATENTSCHRIFT

(11) **EP 0 526 737 B1**
(45) Veröffentlichungstag und Bekanntmachung des Hinweises auf die Patenterteilung: **15.03.1995**
(21) Anmeldenummer: 92111113.4
(22) Anmeldetag: 01.07.1992
(51) Int. Cl.: C07C 45/00, C07C 49/17

(54) **Verfahren zur Herstellung von Dihydroxyaceton**
Process for the preparation of dihydroxyacetone
Procédé pour la préparation de dihydroxyacétone

(30) Priorität: 09.07.1991 DE 4122669
(43) Veröffentlichungstag der Anmeldung: 10.02.1993
(73) Patentinhaber: BASF Aktiengesellschaft, 67063 Ludwigshafen (DE)
(72) Erfinder: Gehrer, Eugen, Dr., W-6700 Ludwigshafen (DE); Harder, Wolfgang, Dr., W-6940 Weinheim (DE); Knuth, Bernhard, Dr., W-6711 Laumersheim (DE); Vogel, Herbert, Dr., W-6700 Ludwigshafen (DE)

(56) Entgegenhaltungen:
- EP-A- 0 219 317
- EP-A- 0 410 613
- DATABASE WPIL Week 8444, Derwent Publications Ltd., London, GB; AN 84-272030; & JP-A-59 164 746
- JOURNAL OF THE AMERICAN CHEMICAL SOCIETY, Bd. 106, 1984, Gaston, PA, US, Seiten 4829 - 4832; T. MATSUMOTO et al: "Selective Formation of Triose from Formaldehyde Catalyzed by Thiazolium Salt."

## Beschreibung

Die vorliegende Erfindung betrifft ein Verfahren zur Herstellung von Dihydroxyaceton durch die Kondensation von Formaldehyd in flüssiger Phase mit Hilfe eines Thiazoliumylidkatalysators.

Es ist bekannt, daß die Benzoinkondensation von Thiazoliumsalzen, die in 2-Stellung unsubstituiert sind, in Gegenwart einer Base katalysiert wird. Castells et al (Tetrahedron Lett. 21, 4517 (1980)) übertrugen diese Reaktion erstmals auf die Kondensation von Formaldehyd und erhielten dabei eine komplexe Mischung aus Kohlenhydraten. Matsumoto et al (J. Am. Chem. Soc. 106, 4829 (1984)) wandten verbesserte Reaktionsbedingungen auf diese sogenannte "Formoin-Reaktion" an und erhielten relativ selektiv Dihydroxyaceton neben Glycerinaldehyd und höheren Zuckern.

Üblicherweise wird bei der Durchführung derartiger Acyloinkondensationsreaktionen das verwendete Thiazoliumsalz mit Hilfe einer Hilfsbase, beispielsweise Triethylamin, in 2-Stellung des Thiazoliumrings deprotoniert, wobei die katalytisch aktive Thiazoliumspezies, nämlich das Thiazoliumylid und das Salz der Hilfsbase mit dem ursprünglichen Gegenanion des Thiazoliumsalzes, beispielsweise das betreffende Triethylammoniumsalz entsteht. Bei den obenerwähnten Verfahren wurde die Reaktion immer in Gegenwart dieser Ammoniumsalze mit dem Thiazoliumylidkatalysator durchgeführt.

Ein Problem dieser Reaktion ist, daß unter den obengenannten, üblicherweise angewandten Reaktionsbedingungen ein Teil des Dihydroxyacetons zu Glycerinaldehyd isomerisiert, das in seiner Eigenschaft als Aldehyd mit weiterem Formaldehyd unter Bildung höherer Zucker weiterreagieren kann. Die Bildung solcher Zucker ist aber in der Regel unerwünscht und verringert die Ausbeute an Dihydroxyaceton. Auch die direkte Hydroxymethylierung von Dihydroxyaceton zu Tetrose und höheren Zuckern (z. B. Hydroxymethylglycerinaldehyd) findet unter den üblichen Reaktionsbedingungen statt.

In J. Heterocyclic Chem. 23, 715 (1986) wurde gezeigt, daß das Thiazoliumylid auch in Abwesenheit von Basen die Acyloinkondensation von Aldehyden katalysiert. Dabei setzten die Autoren jener Schrift Lösungen katalytisch aktiver Thiazoliumspezies ein, die durch die Behandlung von Thiazoliumsalzen mit basischen Ionenaustauschern erhalten worden waren. Durch Eindampfen der so erhaltenen Lösungen wurden die katalytisch aktiven Thiazoliumverbindungen in amorpher, fester Form erhalten und als solche ebenfalls zur Katalyse der Benzoin- bzw. Formoin-Kondensation eingesetzt. Bei diesem Vorgehen konnte der Umsatz der Formoinkondensation zwar gesteigert werden, die Selektivität ging aber erheblich zurück und es bildeten sich größere Mengen an höheren Zuckern, so daß dieses Verfahren für die Ausführung im technischen Maßstab ungeeignet ist.

Gemäß EP-A 410 613 werden die zur Erzeugung der Thiazoliumylide im überschuß angewandten Basen als auch die bei deren Erzeugung anfallenden Salze dieser Basen vor der Umsetzung der Aldehyde mit dem Thiazoliumylidkatalysator durch Extraktion mit Wasser entfernt. Als nicht mit Wasser mischbare Lösungsmittel werden sowohl bei der Erzeugung der Thiazoliumylide als auch bei der Herstellung des Dihydroxyacetons langkettige Alkohole, wie 2-Ethylhexanol, verwendet. Nach dem Verfahren dieser Schrift können bei der Herstellung von Dihydroxyaceton aus Formaldehyd nur unbefriedigende Ausbeuten von bestenfalls 55 %, bezogen auf eingesetzten Formaldehyd, erzielt werden. Eine derart niedrige Ausbeute ist für eine wirtschaftliche Verwertung des Verfahrens ebenfalls prohibitiv.

Der vorliegenden Erfindung lag somit die Aufgabe zugrunde, ein Verfahren zu finden, das die Thiazoliumylid-katalysierte, selektive Kondensation von Formaldehyd zu Dihydroxyaceton auf wirtschaftliche Weise und ohne die genannten Nachteile der bekannten Verfahren ermöglicht.

Dementsprechend wurde ein Verfahren zur Herstellung von Dihydroxyaceton durch die Kondensation von Formaldehyd in flüssiger Phase mit Hilfe eines Thiazoliumylidkatalysators gefunden, das dadurch gekennzeichnet ist, daß man die Umsetzung in einem polaren, aprotischen Lösungsmittel in Abwesenheit von aciden und basischen Verbindungen durchführt.

Den Anlaß zur vorliegenden Erfindung gab die Erkenntnis, daß sowohl die Isomerisierung von Dihydroxyaceton zu Glycerinaldehyd als auch die direkte Hydroxymethylierung von Dihydroxyaceton zu Tetrose von Säuren und auch von Basen katalysiert wird. Insbesondere ist in diesem Zusammenhang die katalytische Wirkung von "versteckten" Säuren auf diese Nebenreaktionen hervorzuheben, d. h. Verbindungen, die üblicherweise nicht als Säuren betrachtet werden, unter den Bedingungen der Thiazoliumylid-katalysierten Formoin-Reaktion aber als Säuren wirken und die genannten Nebenreaktionen verursachen.

Unter den aciden und basischen Verbindungen, in deren Abwesenheit das erfindungsgemäße Verfahren durchzuführen ist, werden insbesondere solche aciden und basischen Verbindungen verstanden, wie sie bei der Erzeugung der Thiazoliumylide durch die Deprotonierung von Thiazoliumsalzen mittels einer Hilfsbase, insbesondere einem tertiären Amin, zugesetzt werden oder entstehen (s. Gleichung (1)) 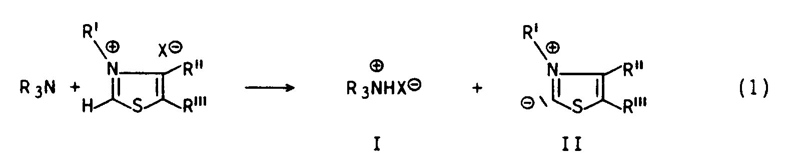
und welche die Isomerisierung des im Zuge der Thiazoliumylid-katalysierten Kondensation von Formaldehyd gebildeten Dihydroxyacetons zu Glycerinaldehyd, gemäß Gleichung (2), 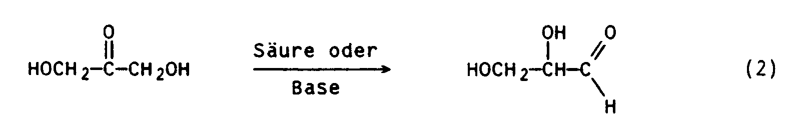
katalysieren. Als acide Verbindungen wären hier insbesondere die Ammonium- oder Amidiniumsalze I beispielhaft zu nennen, die bei Verwendung tertiärer Stickstoffbasen, wie tertiären Aminen oder cyclischen Amidinen (vgl. EP-A 219 317), bei der Erzeugung des Ylides II entstehen. Ebenfalls acide wirkt die durch Wasseranlagerung an den Katalysator entstehende Verbindung der Formel III 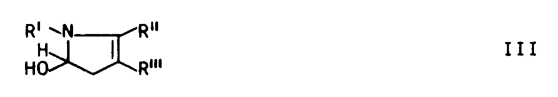
in deren Anwesenheit eine massive Bildung höherer Zucker beobachtet wird.

Als basische Verbindungen wären all diejenigen Verbindungen zu nennen, die geeignet sind, das Thiazoliumsalz zum Thiazoliumylid zu deprotonieren und zu diesem Zwecke letztendlich auch, gegebenenfalls im überschuß bezüglich des Thiazoliumsalzes, eingesetzt werden, deren Anwesenheit bei der anschließenden Thiazoliumylid-katalysierten Formoin-Reaktion aber die Isomerisierung und Homologisierung des Dihydroxyacetons zu Glycerinaldehyd bzw. Tetrose bewirkt.

Aus diesem Grunde sollte sowohl bei der Erzeugung der Thiazoliumylide, als auch bei der Durchführung der Formoin-Reaktion in Abwesenheit von Wasser gearbeitet werden. Zur Vermeidung eines nachteiligen Einflusses eingeschleppter Wasserspuren auf die Katalysatorherstellung als auch auf das Ergebnis der Formoin-Reaktion kann der Reaktionsmischung zur Herstellung der Thiazoliumylidkatalysatoren als auch bei der Formoin-Reaktion vorteilhaft ein Molekularsieb, beispielsweise Molekularsieb 3 A der Firma Grace, zugesetzt werden, um Restmengen an Wasser aus der Reaktionsmischung abzufangen.

Auch protische Lösungsmittel, wie Alkohole, begünstigen in geringerem Ausmaß die Bildung von höheren Zuckern. Deshalb werden die besten Ergebnisse erzielt, wenn man das erfindungsgemäße Verfahren zur Herstellung von Dihydroxyaceton in wasserfreien, polaren aprotischen Lösungsmitteln vornimmt. Als solche Lösungsmittel sind beispielsweise Ether, wie Tetrahydrofuran, Dioxan und Trioxan, Ethylenglycoldimethylether, Ethylenglycoldiethylether, Diethylenglycoldimethylether, Nitrile wie Acetonitril, Amide, wie N,N-Dimethylformamid, N,N-Diethylformamid, N,N-Dipropylformamid, N,N-Dibutylformamid, Hexamethylphosphorsäuretriamid, Lactame, wie N-Methylpyrrolidon, N-Ethylpyrrolidon, Sulfoxide und Sulfone, wie Dimethylsulfoxid und Sulfolan geeignet. Geringe Alkoholgehalte können in diesen Lösungsmitteln toleriert werden, insbesondere wenn sie auf die Erzeugung des Thiazoliumylidkatalysators zurückzuführen sind, besonders bevorzugt wird aber in Abwesenheit solcher Alkohole gearbeitet.

Als Katalysatoren werden im erfindungsgemäßen Verfahren zweckmäßigerweise Thiazoliumring-haltige Katalysatoren verwendet, die in 2-Stellung des Thiazoliumrings unsubstitutiert sind, beispielsweise Thiazoliumylide der Formel II 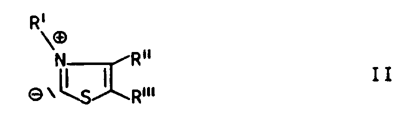
Isoliert man diese Ylide, so erhält man je nach der Art der Erzeugung der Thiazoliumylide Dimere der Formel IIa 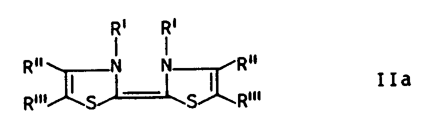
oder Alkoholaddukte der Formel IIb 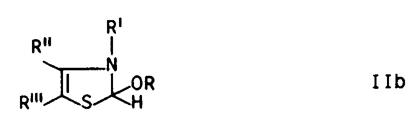
Die Verbindungen der Formel IIa lassen sich vorzugsweise aus Thiazoliumylid-Lösungen isolieren, die durch Deprotonierung der betreffenden Thiazoliumsalze mittels Aminen erhalten worden sind, wohingegen die Verbindungen IIb sich vorzugsweise aus Thiazoliumylid-Lösungen gewinnen lassen, die beispielsweise durch Umsetzung von N-Formyl-N-alkyl-o-mercaptoanilin-Derivaten mit Orthoformiaten erzeugt worden sind. Die Verbindungen IIa und IIb sind stabil, können kristallin erhalten werden und insbesondere IIb als stabile Lagerform für die Ylide II genutzt werden.

Für die katalytische Aktivität der als Katalysatoren verwendeten Thiazoliumverbindungen ist es allein wichtig, daß die daraus hergestellten Thiazoliumylide in 2-Stellung des Thiazoliumrings unsubstituiert sind. Ansonsten können die Thiazoliumylid-Katalysatoren mit beliebigen Substituenten R' , R'' oder R''' substituiert sein. Beispielsweise können Thiazoliumylide der Formel IIc 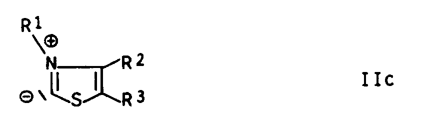
oder Benzothiazoliumylide IId 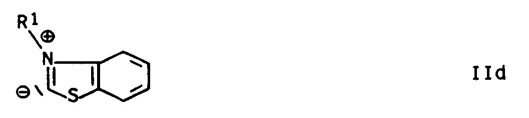
eingesetzt werden, in denen R¹ z. B. für eine C₁- bis C₃₀-Alkylgruppe, vorzugsweise eine C₂- bis C₂₀-Alkylgruppe oder eine Halogenalkylgruppe entsprechender Kohlenstoffzahl, die als Halogenatome vorzugsweise Fluor- oder Chloratome enthalten kann, eine an einen polymeren Träger gebundene C₁- bis C₂₅-Alkylengruppe, eine C₇- bis C₂₀-Aralkylgruppe, vorzugsweise eine C₇- bis C₁₂-Aralkylgruppe, insbesondere die Benzylgruppe oder eine Arylgruppe, wie die Phenyl- oder Naphthylgruppe, stehen kann und in denen die Reste R² und R³ gleich oder verschieden sein können und Wasserstoff, ein Halogenatom, eine C₁- bis C₁₀-Alkylgruppe, vorzugsweise eine C₁- bis C₄-Alkylgruppe, eine C₇- bis C₁₂-Aralkylgruppe, vorzugsweise die Benzylgruppe und/oder eine Phenyl- und/oder Naphthylgruppe bedeuten können. Die Reste R¹, R² und R³ können gewünschtenfalls noch unter den Reaktionsbedingungen inerte Substituenten tragen, wie Alkylgruppen, Alkoxygruppen, Halogenatome, C₂- bis C₁₀-Dialkylaminogruppen, C₁- bis C₁₀-Alkylthiogruppen, Nitrogruppen oder Cyanogruppen. Da die Herstellung von Thiazoliumsalzen, in denen die Reste R¹, R² und R³ solche zusätzlichen Substituenten tragen, im allgemeinen ziemlich aufwendig ist, da in der Regel teure Ausgangsmaterialien verwendet werden müssen, ist die Anwendung der daraus hergestellten Thiazolium- oder Benzothiazoliumylide im erfindungsgemäßen Verfahren aus Kostengründen im allgemeinen weniger bevorzugt. Bevorzugt und vorteilhaft werden im erfindungsgemäßen Verfahren insbesondere Benzothiazoliumylide der Formel IId verwendet. Besonders bevorzugt werden Benzothiazoliumylide mit lipophilen Resten R¹ eingesetzt, insbesondere solche, in denen R¹ ein C₁₀- bis C₂₀-Alkylrest ist. Insbesonders langkettige Reste R¹ können die Lipophilie der Thiazoliumsalze und der daraus hergestellten Thiazoliumylidkatalysatoren und somit deren Löslichkeit in unpolaren organischen Lösungsmitteln erhöhen.

Die Thiazolium- und Benzothiazoliumylide können auch an einem polymeren, organischen oder anorganischen Träger, beispielsweise ein vernetztes Styrol-Divinylbenzol-Harz oder ein Phenol-Formaldehyd-Harz, z. B. über den Rest R¹, angebunden sein, wobei der Rest R¹ eine C₁- bis C₂₅-, vorzugsweise eine C₁- bis C₄-Alkylengruppe sein kann.

Zur Erzeugung der erfindungsgemäßen Katalysatorlösung zur Herstellung von Dihydroxyaceton, in der sich zwar der Thiazoliumylidkatalysator, nicht aber die zu seiner Erzeugung verwendeten Basen und nicht die bei seiner Erzeugung entstehenden aciden Verbindungen enthalten sind, gibt es verschiedenerlei Möglichkeiten.

So können die Thiazolium- und Benzothiazoliumylide in Lösung durch Erhitzen der inneren, betainartigen Thiazolium- und Benzothiazoliumsalze der allgemeinen Formel IV 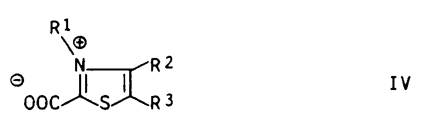
und V 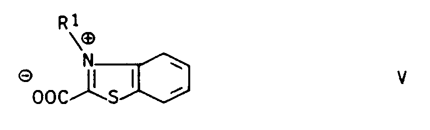
unter Abspaltung von Kohlendioxid erzeugt werden.

Eine andere Möglichkeit besteht in der direkten Synthese von Benzothiazoliumyliden IId durch die Umsetzung der entsprechenden N-Formyl-N-alkyl-o-mercaptoanilin-Derivate VI mit wasserentziehenden Mitteln, wie Orthoameisensäureestern, gemäß Reaktionsgleichung (3) 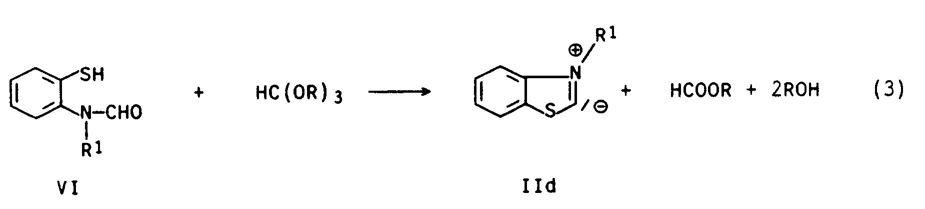
Die Verbindungen VI bilden sich bei der Umsetzung der N-Alkyl-o-mercaptoaniline mit Ameisensäure und deren Derivaten, wie Ameisensäureestern und Orthoameisensäureestern. Die als Ausgangsprodukte zur Herstellung dieser Verbindungen dienenden o-Mercaptoaniline können beispielsweise nach dem Verfahren der DE-PS 367 346 erhalten werden. Die Reste R der Orthoameisensäureester sind vorzugsweise C₁- bis C₄-Alkylgruppen. Die bei der Herstellung der Thiazoliumylide nach diesem Verfahren in geringer Menge bezüglich des Gesamtvolumens der Reaktionsmischung gebildeten Alkohole ROH können bei der Herstellung des Dihydroxyacetons toleriert werden.

Eine andere Methode zur Herstellung der Thiazoliumylide besteht in der Deprotonierung der entsprechenden Thiazoliumring-haltigen Verbindungen, wie denen der Formel VII 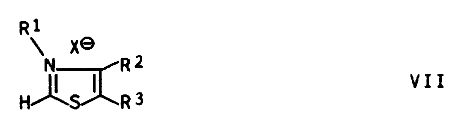
oder der Benzothiazoliumsalze VIII 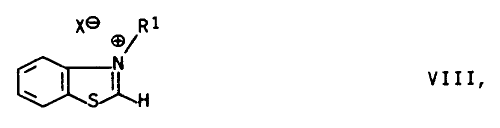
in denen R¹, R² und R³ die obengenannte Bedeutung haben und in denen das Gegenanion X^{⊖} zum Thiazoliumkation beliebig gewählt werden und beispielsweise ein Halogenidanion oder eine Nitrat-, Perchlorat-, Tetrafluoroborat-, Toluolsulfonat-, Methansulfonat-, Benzolsulfonat-, Hydrogensulfat- oder Acetat-Gruppe sein kann, mit Hilfe nichtnucleophiler Basen.

Solche Thiazoliumsalze sind leicht nach dem allgemein anwendbaren Verfahren von Matsumoto et al (J. Am. Chem. Soc. 106, 4829 (1984)) durch die Umsetzung der betreffenden Thiazole oder des Benzothiazols mit Alkylhalogeniden erhältlich. Die betreffenden Thiazole können nach bekannten Methoden (s. z. B. G. Vernin, General Synthetic Methods for Thiazole or Thiazolium Salts, in: Thiazole and its Derivatives (The Chemistry of Heterocyclic Compounds, Vol. 34, part I), Ed.: J.V. Metzger, John Wiley & Sons, New York (1979)), synthetisiert werden. Die Alkylhalogenide sind leicht aus den entsprechenden Fettalkoholen, z. B. durch deren Behandlung mit Halogenwasserstoffsäuren, zugänglich.

Als nichtnucleophile Hilfsbasen zur Erzeugung der Thiazoliumylide können z. B. Alkalimetallalkoxide, insbesondere tertiärer Alkohole, wie tert.-Butanol oder tert.-Amylalkohol, verwendet werden. Dabei bildet sich der entsprechende Alkohol sowie das betreffende Alkalimetallsalz mit dem Gegenanion des Thiazoliumsalzes, welches vorteilhaft vom Reaktionsgemisch abfiltriert wird. Da diese Alkohole nur in Mengen entsprechend der Konzentration des Thiazoliumylid-Katalysators gebildet werden, ist ihre Menge im Vergleich zum Gesamtvolumen des Reaktionsmediums gering, weshalb ihre Anwesenheit im Reaktionsmedium geduldet werden kann.

Bevorzugt werden als nichtnucleophile Hilfsbasen zur Herstellung der Thiazoliumylide aus Thiazoliumsalzen tertiäre Amine mit 3 bis 30 Kohlenstoffatomen oder sterisch gehinderte cyclische Amidine, wie sie z. B. in EP-A 219 317 beschrieben sind, eingesetzt, die aufgrund ihrer Basenstärke in der Lage sind, die betreffenden Thiazolium- bzw. Benzothiazoliumsalze in 2-Stellung des Thiazoliumringes zu deprotonieren.

Geeignete tertiäre Amine sind beispielsweise Trimethylamin, Triethylamin, Tripropylamin, Tributylamin, Ethyldiisopropylamin, Decyldiethylamin, Tridecylamin, Chinuclidin, Diazabicyclo-[2,2,2]-octan, N-Methyl-piperidin, N-Ethylpiperidin, N-Propylpiperidin, N-Butylpiperidin, N,N'-Dimethylpiperazin, N-Methylmorpholin, Dimethylbenzylamin, Dibenzylmethylamin, Benzyldioctylamin, Benzyldiethylamin, Cyclohexyldiethylamin, Dicyclohexylmethylamin, Dicyclohexylethylamin, usw. Besonders bevorzugt wird Triethylamin verwendet. Von den sterisch gehinderten, cyclischen Amidinen werden 1,5-Diazabicyclo-[4,3,0]-non-5-en, 1,8-Diazabicyclo-[5,4,0]-undec-7-en und 1,5,7-Triazabicyclo-[4,4,0]-dec-7-en vorzugsweise eingesetzt.

Es können auch polymere tertiäre Amine als Hilfsbasen verwendet werden, beispielsweise vernetzte Styrol-Divinylbenzol-Harze oder Phenol-Formaldehyd-Harze, die Seitenketten mit tertiären Aminogruppen tragen oder deren Arylgruppen mit Dialkylaminogruppen substituiert sind. Solche polymeren Amine werden üblicherweise als Anionenaustauscher eingesetzt. Bei der Anwendung solcher Anionenaustauscher zur Erzeugung der Thiazoliumylide ist darauf zu achten, daß diese nicht in der OH^{⊖}-Form vorliegen und kein Wasser enthalten.

Zur Vermeidung der beschriebenen Isomerisierungsreaktionen werden die erzeugten Thiazoliumylidkatalysatoren vor ihrer Verwendung zur Herstellung von Dihydroxyaceton von dem bei ihrer Erzeugung gebildeten Salz der Hilfsbase und gegebenenfalls überschüssigen Mengen an eingesetzter Hilfsbase abgetrennt. Zu diesem Zweck kann entweder das Salz der Hilfsbase ausgefällt und vom Thiazoliumylid-Katalysator mechanisch abgetrennt werden oder es kann das gebildete Thiazoliumylid vom in Lösung befindlichen Salz der Hilfsbase durch Kristallisation oder durch Extraktion mit einem wasserfreien, aprotischen Lösungsmittel abgetrennt werden.

Vorteilhaft werden solche Lösungsmittel angewandt, in denen die Thiazoliumylide und das bei der Deprotonierung des Thiazoliumsalzes entstehende Salz der Hilfsbase deutlich voneinander verschiedener Löslichkeit haben. In der Regel löst sich das Thiazoliumylid besser in relativ unpolaren Lösungsmitteln, wie Kohlenwasserstoffen, z.B. Cyclohexan oder Toluol, in Ethern, halogenierten Kohlenwasserstoffen und Ketonen, wohingegen sich das Salz der Hilfsbase leichter in relativ polaren Lösungsmitteln, wie N,N-Dialkylamiden, Lactamen, cyclischen Harnstoffen, Sulfoxiden und Sulfonen löst. Beispielhaft seien als relativ unpolare Lösungsmittel Tetrahydrofuran, Dioxan, Ethylenglycoldimethylether, Ethylenglycoldiethylether, Ethylenglycoldibutylether, Diethylenglycoldimethylether, Diethylenglycoldiethylether, Diethylenglycoldibutylether, Triethylenglycoldimethylether, Dichlormethan, Chloroform, Chlorbenzol, Dichlorbenzol, Aceton, Diethylketon, Methylethylketon, Dipropylketon, Dibutylketon usw. genannt, wohingegen N,N'-Dimethylethylenharnstoff, N,N'-Diethylethylenharnstoff, N,N'-Dibutylethylenharnstoff, N,N'-Dimethyl-1,3-propylenharnstoff, N,N'-Diethyl-1,3-propylenharnstoff, N,N'-Dibutyl-1,3-propylenharnstoff, N-Methylpyrrolidon, N-Ethylpyrrolidon, N-Butylpyrrolidon, N,N-Dimethylformamid, N,N-Diethylformamid, N,N-Dibutylformamid, Hexamethylphosphorsäuretriamid, Dimethylsulfoxid und Sulfolan Beispiele für relativ polare Lösungsmittel sind.

Je nachdem ob sich das Thiazoliumylid oder das bei dessen Erzeugung entstehende Salz der Hilfsbase im verwendeten Lösungsmittel besser löst, kann folgendermaßen vorgegangen werden:
Löst sich das Thiazoliumylid besser im Lösungsmittel als das Salz der Hilfsbase, dies kann z. B. bei Verwendung eines unpolaren Lösungsmittels der Fall sein, so trennt man das ausgefallene Salz der Hilfsbase vom gelösten Thiazoliumylidkatalysator ab, beispielsweise durch Filtration oder Zentrifugation. Vorteilhaft wird bei dieser Vorgehensweise ein Lösungsmittel gewählt, in dem der Thiazoliumylidkatalysator gut und das Salz der Hilfsbase schlecht löslich ist. Bei Verwendung eines solchen Lösungsmittels ist es möglich, daß sich auch das zur Herstellung des Katalysators benötigte Ausgangsmaterial, das Thiazoliumsalz, nicht vollständig löst. In einem solchen Fall kann das im verwendeten Lösungsmittel lösliche Thiazoliumylid aus dem Thiazoliumsalz durch eine Umfällung in Gegenwart der Hilfsbase erzeugt werden, wobei sich schließlich das Satz der Hilfsbase abscheidet.

Die Umsetzung des Thiazoliumsalzes mit der Hilfsbase kann bei Temperaturen von im allgemeinen 0 bis 150°C, vorzugsweise 20 bis 120°C und besonders bevorzugt bei 50 bis 100°C vorgenommen werden. Insbesondere wenn der Thiazoliumylidkatalysator durch eine Umfällung erzeugt wird, ist die Anwendung erhöhter Temperaturen bevorzugt. Das bei der Erzeugung des Thiazoliumylidkatalysators bei dieser Arbeitsweise ausfallende Satz der Hilfsbase kann unmittelbar von der Katalysatorlösung abfiltriert werden, vorteilhaft wird die Katalysatorlösung vom ausgefallenen Satz der Hilfsbase in der Regel erst nach Abkühlung der erhaltenen Suspension auf Raumtemperatur oder gewünschtenfalls auf tiefere Temperaturen abgetrennt.

Die auf diese Weise erhaltene Katalysatorlösung kann direkt zur erfindungsgemäßen Herstellung von Dihydroxyaceton eingesetzt werden.

Löst sich hingegen das Satz der Hilfsbase besser im verwendeten Lösungsmittel als das Thiazoliumylid, dies kann z. B. bei Verwendung von polaren Lösungsmitteln der Fall sein, so läßt sich des Thiazoliumylid durch Kristallisation in sehr reiner Form erhalten und kann auf herkömmliche Weise, beispielsweise durch Filtration oder Zentrifugation, vom gelösten Salz der Hilfsbase abgetrennt werden.

Die Umsetzung der Hilfsbase mit dem Thiazoliumsalz kann auch bei dieser Verfahrensweise bei den bereits erwähnten Temperaturen vorgenommen werden. Zur Kristallisation des Thiazoliumylidkatalysators wird die Reaktionslösung zweckmäßigerweise auf Raumtemperatur oder auf tiefere Temperaturen abgekühlt. Bei der Anwendung von niedrigeren Temperaturen als Raumtemperatur zur Kristallisation des Thiazoliumylidkatalysators sollte bei dieser Arbeitsweise ferner darauf geachtet werden, daß das Salz der Hilfsbase unter diesen Bedingungen nicht mit auskristallisiert.

Zur Erzeugung des Thiazoliumylids werden die Thiazoliumsalze mit einer äquivalenten oder überschüssigen Menge einer Hilfsbase umgesetzt. Selbstverständlich können auch höhere Hilfsbasenüberschüsse angewandt werden. Mit überschüssigen Mengen an Hilfsbase kann insbesondere dann vorteilhaft gearbeitet werden, wenn das Thiazoliumylid durch Kristallisation von der Lösung der Hilfsbase und deren Salzen abgetrennt wird. Bei der Herstellung von Thiazoliumylidkatalysator-Lösungen über die Umsetzung der Thiazoliumsalze mit der Hilfsbase und die anschließende Entfernung des kristallinen Salzes der Hilfsbase ist es hingegen zweckmäßig, stöchiometrische Mengen der Hilfsbase einzusetzen oder die überschüssige Base abzudestillieren.

Der auf diese Weise erhaltene, kristalline oder gelöste Thiazoliumylidkatalysator kann sogleich zur erfindungsgemäßen Herstellung von Dihydroxyaceton verwendet werden, es ist aber auch möglich ihn zu lagern. Die Lagerung des Katalysators wird zweckmäßigerweise, ebenso wie seine Herstellung als auch die Herstellung des Dihydroxyacetons, unter einer Inertgasatmosphäre vorgenommen. Als Inertgase eignen sich beispielsweise Stickstoff oder Argon.

Falls Anionenaustauscherharze zur Erzeugung der Thiazoliumylide verwendet werden, so können diese nach der Deprotonierung der Thiazoliumsalze von der Ylid-Lösung abfiltriert werden oder aber es kann die Lösung des verwendeten Thiazoliumsalzes zur Erzeugung des Ylides über den in einer Festbettanordnung angebrachten Anionenaustauscher geleitet werden.

Zur Herstellung des Dihydroxyacetons wird gasförmiger Formaldehyd oder Paraformaldehyd in ein geeignetes, polares aprotisches Lösungsmittel, beispielsweise N,N-Dimethylformamid, N,N-Diethylformamid, N,N-Dipropylformamid, N,N-Dibutylformamid, N-Methylpyrrolidon, Acetonitril, Dimethylsulfoxid, Sulfolan, Tetrahydrofuran, Dioxan, Ethylenglycoldimethylether oder Diethylenglycoldimethylether, eingeleitet und auf an sich herkömmliche Weise (vgl. J. Heterocyclic Chem. 23, 715 (1986), EP-A 306 215) mit der Lösung des reinen, d. h. des von den genannten aciden und basischen Verbindungen befreiten Thiazoliumylids in flüssiger Phase, in einem wasserfreien, polaren aprotischen Lösungsmittel umgesetzt. Als Lösungsmittel können beispielsweise die zur Herstellung der Thiazoliumylidkatalysatoren verwendeten Lösungsmittel verwendet werden, es können aber auch von diesen verschiedene Lösungsmittel eingesetzt werden, soweit sie sich unter den Reaktionsbedingungen inert verhalten.

Für das Molverhältnis von Formaldehyd zu Thiazoliumylidkatalysator gibt es praktisch keine Beschränkung nach oben, zweckmäßigerweise kann das Molverhältnis 3 bis 10 000 : 1 betragen.

Die Kondensationsreaktion wird im allgemeinen bei Temperaturen von 20 bis 200°C, vorzugsweise bei 50 bis 150°C und besonders bevorzugt bei 80 bis 120°C ausgeführt. Es ist möglich die Reaktion bei Atmosphärendruck, unter erhöhtem Druck, beispielsweise unter dem Eigendruck des Reaktionssystems oder unter reduziertem Druck durchzuführen.

Das erfindungsgemäße Verfahren ermöglicht die wirtschaftliche Herstellung von Dihydroxyacetonlösungen in guten Ausbeuten und Selektivitäten.

Dihydroxyaceton wird als Bräunungsmittel in kosmetischen Bräunungscremes verwendet und dient darüber hinaus als Ausgangsmaterial zur Herstellung von Glycerin (vgl. EP-A 306 215).

### Beispiele

Die Ausbeute an Dihydroxyaceton wurde in allen Beispielen, nach Bildung des Oxims und Silylierung desselben, gaschromatographisch ermittelt. Alle verwendeten Chemikalien waren wasserfrei. Als zusätzlicher Wasserfänger wurde bei allen Umsetzungen Molekularsieb 3A (Grace Typ 564) zugesetzt.

### Vergleichsbeispiel

Dieses Beispiel beschreibt eine konventionelle Durchführung der Kondensationsreaktion unter optimierten Reaktionsbedingungen.

4,35 g (0,01 mol) N-Hexadecylbenzothiazoliumbromid, 1,01 g Triethylamin, 10,0 g Diethylenglykoldiethylether, 10,0 g Paraformaldehyd, 0,8 g Molekularsieb und 78,0 g Dimethylformamid wurden 30 min lang bei 100°C gerührt. Die Reaktionsmischung enthielt laut gaschromatographischer Analyse 7,76 Gew.-% Dihydroxyaceton, entsprechend einer Ausbeute von 80,7 %. Dieses Ergebnis steht in gutem Einklang mit den Beispielen von Matsumoto et al (J. Am. Chem. Soc. 106, 4829 (1984)).

Herstellung einer Lösung des Thiazoliumylidkatalysators durch Umfällung:
17,48 g (0,04 mol) N-Hexadecyl-benzothiazoliumbromid, 40 g Diethylenglycoldiethylether und 4,08 g (0,04 mol) Triethylamin wurden unter Stickstoff auf 80°C erwärmt und solange gerührt, bis die Umfällung vollständig und eine einheitliche Suspension entstanden war. Nach dem Abkühlen wurde das ausgefallene Triethylammoniumbromid abfiltriert. Das Filtrat wird im folgenden als Katalysatorlösung bezeichnet.

### Beispiel 1 (erfindungsgemäß)

Dieses Beispiel belegt den Ausbeutesprung, den man durch die Abtrennung der zur Base konjugierten Säure erzielen kann.

11,65 g Katalysatorlösung, 10,19 g Paraformaldehyd, 0,8 g Molekularsieb und 78,0 g Dimethylformamid wurden 30 min lang bei 100°C gerührt. Die Analyse der Reaktionsmischung ergab einen Gehalt von 9,22 Gew.-% Dihydroxyaceton, entsprechend einer Ausbeute von 92,2 %.

### Beispiel 2

Dieses Beispiel zeigt, daß ein überschuß an Base die Ausbeute senkt.

11,5 g Katalysatorlösung, 10,02 g Paraformaldehyd, 0,8 g Molekularsieb, 78,0 g Dimethylformamid und 0,36 g Triethylamin wurden bei 100°C 30 min gerührt. Der gaschromatographisch ermittelte Gehalt der Reaktionsmischung an Dihydroxyaceton betrug 7,55 Gew.-%, entsprechend einer Ausbeute von 75,5 %.

### Beispiel 3

Dieses Beispiel belegt den nachteiligen Einfluß des Salzes der Hilfsbase auf die Ausbeute an Dihydroxyaceton.

11,2 g Katalysatorlösung, 10,0 g Paraformaldehyd, 0,8 g Molekularsieb, 78,0 g Dimethylformamid und 0,61 g Triethylammoniumbromid wurden bei 100°C 30 min lang gerührt. Die Reaktionsmischung enthielt 6,39 g Dihydroxyaceton, entsprechend einer Ausbeute von 63,9 %.

### Isolierung des Thiazoliumylidkatalysators

25,7 g (58,5 mmol) N-Hexadecylbenzothiazoliumbromid wurden in 500 ml Dimethylformamid zusammen mit 6,2 g (61,5 mmol) Triethylamin in der Wärme gelöst. Anschließend wurde die Reaktionsmischung auf Raumtemperatur abgekühlt und der Niederschlag, das Thiazoliumylid, abfiltriert. Sämtliche Maßnahmen wurden unter einer Stickstoffatmosphäre ausgeführt.

### Beispiel 4

Dieses Beispiel belegt, daß man die Reaktion mit dem reinen Thiazoliumylid durchführen kann und dabei keine Base benötigt. Unter diesen Bedingungen wird sogar eine besonders hohe Ausbeute erzielt, da die Bildung von Neben- und Folgeprodukten weitgehend unterdrückt wird.

17,0 g Dimethylformamid, 0,96 g (2,67 mmol) des kristallisierten Thiazoliumylidkatalysators, 2,0 g Paraformaldehyd und 0,2 g Molsieb wurden 60 min lang bei 100°C gerührt. Während dieser Zeit bildete sich Dihydroxyaceton in einer Ausbeute von 93,8 %.

### Beispiel 5

Zum Vergleich mit Beispiel 4 wurde ein Reaktionsansatz ohne vorherige Abtrennung des Triethylammoniumbromides umgesetzt.

17,0 g Dimethylformamid, 1,17 g (2,87 mmol) N-Hexdecylbenzothiazoliumbromid, 0,27 g (2,67 mmol) Triethylamin und 0,2 g Molekularsieb wurden 60 min lang bei 100°C gerührt. Während dieser Zeit bildete sich Dihydroxyaceton in einer Ausbeute von 79,5 %.

### Beispiel 6

Dieses Beispiel belegt, daß man den Katalysator unter Reaktionsbedingungen in situ, ohne die Verwendung einer Hilfsbase und ohne die Anwesenheit eines Anions aus einer Vorstufe erzeugen kann.

1,0 g (2,86 mmol) o-Mercapto-N-formyl-N-hexadecylanilin, 0,42 g (2,86 mmol) Triethylorthoformiat, 2 g Paraformaldehyd, 0,2 g Molsieb und 16,5 g Dimethylformamid wurden 4 Stunden lang auf 100°C erwärmt. Während dieser Zeit bildete sich Dihydroxyaceton in einer Ausbeute von 56 %.

## Patentansprüche

1. Verfahren zur Herstellung von Dihydroxyaceton durch die Kondensation von Formaldehyd in flüssiger Phase mit Hilfe eines Thiazoliumylidkatalysators, dadurch gekennzeichnet, daß man die Umsetzung in einem polaren, aprotischen Lösungsmittel in Abwesenheit von aciden und basischen Verbindungen durchführt.

2. Verfahren nach Anspruch 1, dadurch gekennzeichnet, daß man das Thiazoliumylid in einem unpolaren Lösungsmittel erzeugt, in dem das bei der Erzeugung des Thiazoliumylids durch die Umsetzung eines Thiazoliumsalzes mit einer Hilfsbase entstehende Salz der Hilfsbase unlöslich ist und das ausgefallene Salz der Hilfsbase von der so erhaltenen Katalysatorlösung mechanisch abtrennt und den so erhaltenen Katalysator zur Herstellung des Dihydroxyacetons einsetzt.

3. Verfahren nach Anspruch 1, dadurch gekennzeichnet, daß man das Thiazoliumylid in einem polaren Lösungsmittel erzeugt und von dem bei der Erzeugung des Thiazoliumylids aus einem Thiazoliumsalz mit einer Hilfsbase entstandenen Salz der Hilfsbase durch Kristallisation abtrennt und den so erhaltenen Thiazoliumylidkatalysator zur Herstellung des Dihydroxyacetons einsetzt.

4. Verfahren nach Anspruch 1, dadurch gekennzeichnet, daß der Thiazoliumylidkatalysator in der Reaktionsmischung aus einem N-Formyl-N-alkyl-o-mercaptoanilin erzeugt wird.

5. Verfahren nach Anspruch 1, dadurch gekennzeichnet, daß der Thiazoliumylidkatalysator in der Reaktionsmischung aus einem N-Formyl-N-alkyl-o-mercaptoanilin in Gegenwart eines wasserentziehenden Mittels erzeugt wird.

## Claims

1. A process for preparing dihydroxyacetone by condensation of formaldehyde in liquid phase with the aid of a thiazolium ylide catalyst, which comprises carrying out the reaction in a polar, aprotic solvent in the absence of acidic and basic compounds.

2. A process as claimed in claim 1, wherein the thiazolium ylide is generated in a non-polar solvent in which the salt which is produced from the base used in the generation of the thiazolium ylide by reaction of a thiazolium salt with a base is insoluble, and the precipitated salt of the base is separated mechanically from the resulting catalyst solution, and the resulting catalyst is used to prepare the dihydroxyacetone.

3. A process as claimed in claim 1, wherein the thiazolium ylide is generated in a polar solvent and separated by crystallization from the salt which is produced from the base used in the generation of the thiazolium ylide from a thiazolium salt with a base, and the resulting thiazolium ylide catalyst is used to prepare the dihydroxyacetone.

4. A process as claimed in claim 1, wherein the thiazolium ylide catalyst is generated in the reaction mixture from an N-formyl-N-alkyl-o-mercaptoaniline.

5. A process as claimed in claim 1, wherein the thiazolium ylide catalyst is generated in the reaction mixture from an N-formyl-N-alkyl-o-mercaptoaniline in the presence of a dehydrating agent.

## Revendications

1. Procédé de préparation de la dihydroxyacétone par la condensation du formaldéhyde en phase liquide à l'aide d'un catalyseur à base d'ylure de thiazolium, caractérisé en ce que l'on entreprend la réaction dans un solvant polaire et aprotique en l'absence de composés acides et basiques.

2. Procédé suivant la revendication 1, caractérisé en ce que l'on obtient l'ylure de thiazolium dans un solvant apolaire, dans lequel le sel de la base adjuvante qui se forme au cours de l'obtention de l'ylure de thiazolium par la réaction d'un sel de thiazolium avec une base adjuvante est insoluble et on sépare mécaniquement le sel de la base adjuvante précipité d'avec la solution de catalyseur ainsi obtenue et on utilise le catalyseur ainsi réalisé pour la préparation de la dihydroxyacétone.

3. Procédé suivant la revendication 1, caractérisé en ce que l'on obtient l'ylure de thiazolium dans un solvant polaire et on le sépare du sel de la base adjuvante qui se forme au cours de l'obtention de l'ylure de thiazolium à partir d'un sel de thiazolium avec une base adjuvante, par cristallisation et on utilise le catalyseur à base d'ylure de thiazolium ainsi formé pour la préparation de la dihydroxyacétone.

4. Procédé suivant la revendication 1, caractérisé en ce que l'on obtient le catalyseur à base d'ylure de thiazolium dans le mélange réactionnel à partir d'une N-formyl-N-alkyl-o-mercaptoaniline.

5. Procédé suivant la revendication 1, caractérisé en ce que l'on obtient le catalyseur à base d'ylure de thiazolium dans le mélange réactionnel à partir d'une N-formyl-N-alkyl-o-mercaptaoaniline en présence d'un agent absorbant l'eau.
